# EUROPEAN PATENT APPLICATION

(11) **EP 3 662 924 A1**
(43) Date of publication of application: **10.06.2020**
(21) Application number: 19209703.8
(22) Date of filing: 06.03.2015
(51) Int. Cl.: A61K 38/19, A61K 31/665, A61K 9/00, A61K 31/407, A61K 45/06, A61P 11/00, A61P 31/04

(54) **COMPOSITIONS FOR TREATING LUNG INFECTIONS BY AIRWAY ADMINISTRATION**

(30) Priority: 07.03.2014 DK PA201470113
(62) Divisional of application: 15708512.7
(71) Applicant: Reponex Pharmaceuticals A/S, 2970 Hørsholm (DK)
(72) Inventor: HESLET, Lars, "deceased" (DK); UTTENTHAL, Lars Otto, 28009 Madrid (ES)
(74) Representative: Zacco Denmark A/S

(57) **Abstract**

The present invention provides compositions comprising as an essential feature granulocyte-macrophage colony-stimulating factor (GM-CSF) together with fosfomycin for the treatment of bacterial lung infections by administration via the airways.

SEQUENCE LISTING

<110>  Reponex  ApS

<120>  Compositions for treating lung infections by airway administration


<141>  2015-03-06

<160>  1

<170>  PatentIn version 3.5

<210>  1
<211>  144
<212>  PRT
<213>  Homo Sapiens

<400>  1

Met Trp Leu Gln Ser Leu Leu Leu Leu Gly Thr Val Ala Cys Ser Ile
1               5                   10                  15


Ser Ala Pro Ala Arg Ser Pro Ser Pro Ser Thr Gln Pro Trp Glu His
            20                  25                  30


Val Asn Ala Ile Gln Glu Ala Arg Arg Leu Leu Asn Leu Ser Arg Asp
        35                  40                  45


Thr Ala Ala Glu Met Asn Glu Thr Val Glu Val Ile Ser Glu Met Phe
    50                  55                  60


Asp Leu Gln Glu Pro Thr Cys Leu Gln Thr Arg Leu Glu Leu Tyr Lys
65                  70                  75                  80


Gln Gly Leu Arg Gly Ser Leu Thr Lys Leu Lys Gly Pro Leu Thr Met
                85                  90                  95


Met Ala Ser His Tyr Lys Gln His Cys Pro Pro Thr Pro Glu Thr Ser
            100                 105                 110


Cys Ala Thr Gln Ile Ile Thr Phe Glu Ser Phe Lys Glu Asn Leu Lys
        115                 120                 125


Asp Phe Leu Leu Val Ile Pro Phe Asp Cys Trp Glu Pro Val Gln Glu
    130                 135                 140


<210>  2
<211>  144
<212>  PRT
<213>  Homo Sapiens

<400>  2

## Description

### Field of invention

The present invention provides compositions comprising as their essential ingredients granulocyte-macrophage colony-stimulating factor (GM-CSF) and the broad-spectrum antibiotic fosfomycin, for the treatment of severe acute pneumonia and chronic lung infections, especially chronic lung infections and infections with antibiotic-resistant bacteria, by inhalation or other form of airway administration. As such, it is relevant to the fields of respiratory or chest medicine, infectious diseases, intensive care and internal medicine in general.

### Background of the invention

### Bacterial lung infections

Bacterial lung infections are a major source of disease in both the developed and the developing world and may affect any individual at some time during life, even individuals without known predisposing factors. The effective treatment of such infections is a major concern to all specialties of internal medicine, intensive care medicine and post-operative surgical care, and is the subject of numerous scientific papers, reviews and chapters in medical textbooks.

In every case, the appearance of a clinical bacterial lung infection represents at least an initial failure of the natural host defenses of the airways to clear the infective organisms. These natural defense mechanisms of the airways consist of many cooperating features.

### The primary host defense mechanism

One of the most important host defense mechanism encompasses the local expression of GM-CSF in the alveoli and peripheral airways.

Normally the bacteria induce a local effect, in such a way that the resting macrophages in the distal airways express GM-CSF when the bacteria are approaching them. First they start to produce GM-CSF in order to activate the neighboring macrophages by an autocrine mechanism. Secondly, the macrophages, after activation, recruit the peripheral polymorphonuclear neutrophils via the expression of IL-2.

This is a complex chain of events that results in the conversion of the resting macrophages into fully developed dendritic cells, and there are many issues which may derail the process to make the killing of the bacteria less effective.

### The secondary host defense mechanisms

There are several supporting mechanisms, such as mucus secretion and clearance of mucus by ciliary action, the secretion of antibacterial substances such as neutrophil gelatinase-associated lipocalin (NGAL) to sequestrate bacterial siderophores and thus deprive bacteria of the iron that is essential for their growth, the appearance of neutrophils and alveolar macrophages to kill and clear bacteria by their innate immune functions, followed by (if the infection persists, or the infective organism has been experienced before), by the specific mechanisms, cellular and humoral, of acquired immunity.

Some of these mechanisms deal with removing bacterial products after the bacterial killing has taken place. However, the main issue here is that the removal of bacteria has a limited quantitative effect if the killing is defective and the clearing system is overloaded, which may cause an overwhelming inflammatory response and thus lead to mortality.

### The hindrances of the defective host defense

Many congenital and acquired abnormalities can compromise the complex steps of the natural defense mechanisms in the airways.

In ***chronic airways infections**,* among the best-known congenital conditions that hinder the pulmonary host defense mechanisms is *cystic fibrosis,* in which the defect in production of mucus of normal viscosity and the failure to clear the viscous mucus impair the clearance of bacteria from the airways and lead to long-term anatomical changes in the bronchi and bronchioles (Heslet et al. 2012). In addition, the absence of the cystic fibrosis transmembrane conductance regulator chloride channel (CFTR) which lies at the root of the disease, affects not only mucus secretion, but also the adequate function of the neutrophils and alveolar macrophages.

The dilatation of the bronchi attributed to the destruction of elastic tissue in the bronchial wall by an excess of elastase, released from neutrophils in response to chronic infection, results in *bronchiectasis,* with further impairment of mucus clearance.

A further condition is congenital alpha1-antitrypsin (AAT) deficiency, which may lead to or contribute to chronic bronchitis and emphysema with bronchiectasis. It is proposed that deficiency of this protease inhibitor leaves the action of proteolytic enzymes on the airways epithelium unopposed, so that the resulting anatomical and functional changes impair the natural host defenses and lead to chronic lung infections.

Among the numerous acquired abnormalities that compromise the natural host defense mechanisms in the airways is the occurrence of severe illness, including severe systemic infections, trauma, whether non-surgical or surgical in origin, major inflammatory diseases, and indeed any of a very large number of diseases known to adversely affect innate and/or acquired immunity in general. In addition, respiratory complications of severe illness and shock, such as acute respiratory distress syndrome, will in themselves compromise the natural host defense mechanisms of the lungs and predispose to bacterial infection. There are also iatrogenic causes of reduced host defense mechanisms in the lungs, such as immunosuppressive or cytotoxic treatments.

In treating severe bacterial lung infections, and perhaps especially chronic bacterial lung infections in individuals with the predisposing conditions mentioned above, it is the purpose of the present disclosure to propose that that two lines of treatment should be used to achieve an optimal curative or ameliorating effect: delivering an agent to enhance the natural host defense mechanisms that have proved to be inadequate or are known to be defective, and delivering an appropriate antibacterial agent or agents to reduce or eliminate the infective load. Both types of agent are to be delivered to the site where their action is required.

### Enhancing pulmonary host defense against bacterial infection by means of granulocyte-macrophage colony-stimulating factor (GM-CSF)

The use of GM-CSF to enhance pulmonary host defense in general has been disclosed in WO2008/052567, to which reference is hereby made.

GM-CSF is a member of the family of colony-stimulating factors (CSFs), which are glycoproteins that stimulate the proliferation and maturation of hematopoietic progenitors and enhance the functional activity of mature effector cells. In brief, at the level of the immature cells, CSF's ensure the self-renewal of the staminal pool and activate the first stage of hematopoietic differentiation. In the subsequent stage, when cell proliferation is associated with a progressive acquisition of the characteristics of the mature cells, they enormously enhance the number of differentiating cells. In the terminal stage, they stimulate the circulation and the activation of mature cells.

Mature GM-CSF is a monomeric protein of 127 amino-acid residues with several potential glycosylation sites. The variable degree of glycosylation results in a molecular weight range between 14 kDa and 35 kDa. Non-glycosylated and glycosylated GM-CSF show similar activity *in vitro* (Cebon et al. 1990). The crystallographic analysis of GM-CSF revealed a barrel-shaped structure composed of four short alpha helices (Diederichs et al. 1991). There are two known sequence variants of GM-CSF. The active form of the GM-CSF protein is found extracellularly as a homodimer *in vivo.*

GM-CSF exerts its biological activity by binding to its receptor. The most important sites of GM-CSF receptor (GM-CSF-R) expression are on the cell surface of myeloid cells, such as macrophages types I and II, epithelial cells and endothelial cells, whereas lymphocytes are GM-CSF-R negative. The native receptor is composed of alpha and beta subunits. The alpha subunit imparts ligand specificity and binds GM-CSF with nanomolar affinity. The beta subunit is also part of the interleukin-3 and interleukin-5 receptor complexes and, in association with the GM-CSF-R alpha subunit and GM-CSF, leads to the formation of a complex with picomolar binding affinity (Hayashida et al. 1990). The binding domains on GM-CSF for the receptor have been mapped: GM-CSF interacts with the beta subunit of its receptor via a very restricted region in the first alpha helix of GM-CSF (Shanafelt et al. 1991a, b; Lopez et al. 1991). Binding to the alpha subunit could be mapped to the third alpha helix, helix C, the initial residues of the loop joining helices C and D, and to the carboxyterminal tail of GM-CSF (Brown et al. 1994).

Formation of the GM-CSF trimeric receptor complex leads to the activation of complex signaling cascades involving molecules of the JAK/STAT families, She, Ras, Raf, the MAP kinases, phosphatidylinositol-3-kinase and NFkB, finally leading to the transcription of c-myc, c-fos and c-jun. Activation is mainly induced by the beta subunit of the receptor (Hayashida et al. 1990; Kitamura et al. 1991; Sato et al. 1993). The shared beta subunit is also responsible for the overlapping functions exerted by IL-3, IL-5 and GM-CSF (reviewed by de Groot et al. 1998).

In addition to its stimulating activity on hemopoietic growth and differentiation, GM-CSF acts as a proinflammatory cytokine. Macrophages, e.g. macrophages type I & II and monocytes, as well as neutrophils and eosinophils, are activated by GM-CSF, resulting in the release of other cytokines and chemokines and matrix-degrading proteases, as well as increased expression of HLA and cell adhesion molecules or receptors for CC-chemokines. This in turn leads to increased chemotaxis of inflammatory cells into inflamed tissue.

GM-CSF has been shown to have a positive effect on bacterial lung infections animals. Local delivery of GM-CSF protects mice from lethal pneumococcal pneumonia (Steinwede et al. 2011). Correspondingly, GM-CSF-deficient mice are susceptible to pulmonary group B streptococcal infection (LeVine et al. 2005). Toll-like receptor 4 (TLR4) signaling defective mice show diminished pulmonary alveolar cell expression of GM-CSF, increased lung permeability, cell death and bacteremia after experimental pulmonary infection with *Klebsiella pneumoniae,* which is reversed by intratracheal administration of GM-CSF (Standiford et al. 2012). The beneficial effects of GM-CSF in experimental pulmonary infection are discussed by Quinton (2012).

In chronic lung infections with Gram-negative bacteria, such as occur in patients with cystic fibrosis or bronchiectasis, alveolar macrophage function may be impaired because of a T helper cell type 2 (TH2)-dominated alveolar inflammation with CD20+ T-cell activation. This is associated with tolerance of the infective or colonizing organisms and hence a poor prognosis (Moser et al. 2000; Hartl et al. 2005). It is an important effect of locally applied GM-CSF to promote the transformation of this tolerant alveolar environment to one dominated by the normal T helper cell type 1 (TH1)-dominated response with CD8+ and CD16+ T-cell activation. This is associated with more effective bacterial clearance and a better prognosis (Moser et al. 2002). Increased eradication of e.g. *Pseudomonas aeruginosa* in chronically infected patients results in improved quality of life (Fuschillo 2008). The subject has been reviewed by Heslet L et al. (2012).

### Choice of antibacterial agent for bacterial lung infections.

As pointed out earlier a prerequisite for the successful treatment of acute pneumonia and chronic lung infection or colonization is to treat the case by concomitantly
*1. Upgrading or ensuring that the pulmonary host defense is optima, and*
*2. Ensuring that bacterial growth is kept to a minimum or preferably that the infective bacteria are killed.*
This latter is determined by the quality of the inhaled antibiotic or antibacterial agent.

The antibacterial agent to be administered together with GM-CSF in the treatment of bacterial lung infections must evidently have the antibacterial spectrum of activity to be effective against the most commonly occurring pathogens in severe and chronic lung infections. As the innate pulmonary mechanisms for killing bacteria may be impaired, it is preferable that the agent be bactericidal rather than bacteriostatic. It should also be, as far as possible, non-toxic to the lung tissue and the effector cells, such as neutrophils and macrophages, of the innate pulmonary defense. It must capable of being administered to give an effective local concentration to kill the bacteria, estimated to be as much as 25 times the minimum inhibitory concentration (MIC) determined *in vitro,* because of the protective effect of mucus on bacterial survival.

### Choice of antibacterial agents in acute pneumonia

The primary choice of antibiotic or antibacterial agent is not based on surveillance culture, but on empirical grounds. The present evidence-based medicinal principle is based on the "worst case scenario'. There is no second chance to alter the antibacterial spectrum. The principle is to treat with broad-spectrum antibacterial agents and subsequently use narrow-spectrum antibacterial agents.

The main issue is that in spite of a significantly reduced mortality after the introduction of this treatment paradigm, the mortality rate is still unacceptably high.

The inventors of the present invention have discovered that the missing link here is a defective pulmonary host defense.

There are in principle many causative issues in play in this respect. Among these is the administration of oxygen, resulting in a high FiO₂. This known to reduce GM-CSF expression by the alveolar macrophages, implying a correspondingly reduced pulmonary host defense (Baleeiro et al. 2006). By the inhalation of GM-CSF, it is possible to restore the host defense in alveoli and small airways. While evidence-based use of broad-spectrum antibiotics has reduced mortality significantly, the mortality remains unacceptably high.

### Treatment of chronic infection and/or colonization

In chronic bacterial infections of the lower respiratory tract, a different treatment paradigm from that applied to acute pneumonia can be adopted. This is based on surveillance culture, classically by the culture of sputum or brush specimens obtained by bronchoscopy. However, this only detects those bacteria that can readily be cultured and has been supplemented in recent years by molecular culture-independent techniques, such as the analysis of PCR amplicons of 16S rRNA genes, to establish the "respiratory microbiome" in health and in different types of chronic lung infection. In health, 90-97% percent of individuals have no bacteria in the lower respiratory tract that can be detected by conventional culture. However, microbiomics reveal the presence of low levels of genera such as *Streptococcus* (a-hemolytic and occasionally *S. pneumonia*), occasionally *Haemophilus influenzae,* and anaerobic genera such as *Prevotella, Fusobacterium* and *Veillonella.* In chronic obstructive pulmonary disease (COPD), mild to moderate cases show *H. influenzae, S. pneumoniae, Moraxella catarrhalis, H. parainfluenzae* and *Staphylococcus aureus;* in moderate to severe cases and in exacerbations, *Pseudomonas aeruginosa* enters the picture (Beasley et al. 2012). In cystic fibrosis, the dominant microbiological etiology of the chronic infections is *P. aeruginosa, Staphylococcus aureus, Burkholderia cepacia* complex, *Achromobacter xylosoxidans* and *Mycobacterium abscessus*/*chelonae* (Heslet et al. 2012), to which may be added the occasional occurrence of *Stenotrophomonas maltophilia, Escherichia coli* and bacteria characteristic of the upper respiratory tract (Lim et al. 2014). In bronchiectasis, *P. aeruginosa, H. influenzae* and *Streptococcus pneumoniae,* as well as *Veillonella, Prevotella* and *Neisseria* species have been reported (Rogers et al. 2013).

### Fosfomycin

Fosfomycin is the international non-proprietary name of a broad-spectrum antibiotic isolated and characterized in 1969 from *Streptomyces fradiae* strains under the name phosphomycin or phosphonomycin (Hendlin et al., 1969). Its structure was determined to be (-)(IR, 2S)-1,2-epoxypropylphosphonic acid (Christensen et al. 1969), with the systematic (IUPAC) name [(2R,3S)-3-methyloxiran-2-yl]phosphonic acid and a formula weight of 138.1 Da. Fosfomycin is bactericidal and inhibits bacterial cell wall biosynthesis by inactivating the enzyme UDP-N-acetylglucosamine-3-enolpyruvyltransferase, also known as MurA (Brown et al. 1995). This enzyme catalyzes the committed step in peptidoglycan biosynthesis, the ligation of phosphoenolpyruvate to the 3'-hydroxyl group of UDP-N-acetylglucosamine to form N-acetylmuramic acid. Fosfomycin is a phosphoenolpyruvate analogue that inhibits MurA by alkylating an active site cysteine residue. The antibiotic enters the bacterial cell via the glycerophosphate transporter.

Given this mechanism of action, fosfomycin has a broad bactericidal spectrum, being active against aerobic genera such as *Staphylococcus, Streptococcus, Neisseria, Escherichia, Proteus* (indole-negative), *Serratia, Salmonella, Shigella, Pseudomonas, Haemophilus, Vibrio,* and *Klebsiella pneumoniae, Proteus vulgaris, Proteus mirabilis,* being less active against *Moraxella catarrhalis, Burkholderia cepacia, Mycobacterium spp.* and *Stenotrophomonas maltophilia* (Gobernado 2003). It is known to be active against the anaerobic genera *Veillonella* and *Fusobacterium,* and moderately active against *Prevotella.* Fosfomycin is active against methicillin-resistant *Staphylococcus aureus* and extended spectrum β-lactamase (ESBL) enterobacteria. It will be seen that this spectrum of activity comprises a large number of the genera and species in the respiratory microbiomes of chronic lung infections, and possibly provides the widest cover against these microbiomes of any single antibiotic.

There is a low prevalence of bacterial resistance to fosfomycin in the community, and studies of the prevalence of resistant bacteria after the introduction of fosfomycin have shown either no increase or only a modest increase in the prevalence of resistant organisms. However, prolonged exposure to the antibiotic may enable bacteria to evolve resistance by selection of mutants that lack the glycerophosphate transporter pathway. Alternative mechanisms of resistance involve the loss of the inducible hexose phosphate transporter, a Cys-Asp mutation in MurAS, or acquistion of plasmids coding for the fosfomycin inactivating enzymes fosA and fosB (in addition to the chromosomal fosX in *Listeria monocytogenes*). The mutant strains may, however, also show reduced pathogenicity (Karageorgopoulos et al. 2012). This may explain why the emergence of bacterial resistance is seen on prolonged exposure *in vitro,* but much less frequently *in vivo.* The appearance of resistant bacterial strains in controlled clinical trials of orally or intravenously administered fosfomycin has been 3.0% overall, with a maximum of 15% for *Pseudomonas aeruginosa.* In general, fosfomycin is seen to be a valuable addition to the therapeutic armament against multidrug-resistant organisms. There is no experience with the development of resistance to fosfomycin during treatment of bacterially infected chronic ulcers.

Fosfomycin has proved to be remarkably non-toxic to mammalian cells and organs, despite fosfomycin disodium being used at intravenous doses of up to 0.5 g/kg/day in human patients. Here the limiting factor is overload with the counter-ion rather than any toxic effect of the antibiotic. Indeed, fosfomycin has been found to exert a protective effect against the toxic action of other antibiotics, immunosuppressive or chemotherapeutic agents such as aminoglycosides, vancomycin, amphotericin B, polymyxin, ciclosporin and cisplatin (Gobernado 2003). As additional effects it has the capacity to favor phagocytosis and act as an immunomodulator. It is accumulated by polymorphonuclear leukocytes to reach concentrations that are twice those of the extracellular fluid, but does not affect their cellular functions, while exerting a bactericidal effect on *Staphylococcus aureus.* The chief adverse effects are gastric irritation from orally administered fosfomycin disodium, evidence of allergy in the form of transient rashes (0.3% of cases) and eosinophilia (0.2%), and transiently raised liver enzymes (0.3% of cases) (Gobernado 2003).

### The use of fosfomycin in combination with other antibiotics

Fosfomycin shows a considerable synergism in bactericidal effect on a large number of strains of organisms from the susceptible genera mentioned, when used in combination with a large number of antibiotics of the penicillin, cephalosporin, aminoglycoside, macrolide and lincosamide types. While early studies showed a synergistic effect on about 70-100% of tested strains for various antibiotic combinations, subsequent more extensive studies showed synergy rates of 36-74%. The remaining strains showed merely additive effects and an inhibitory effect was only seen in one or two individual antibiotic combinations on an individual bacterial strain (Gobernado 2003). The fact that fosfomycin shows synergy with many individual antibiotics and indeed abrogates the toxicity of many other antibiotics, including the nephrotoxicity and ototoxicity of the aminoglycosides, favors the use of fosfomycin in combination with other antibiotics to produce a potent bactericidal action and compensate for any development of fosfomycin resistance during more prolonged treatment.

### Summary of the invention

Accordingly, the present invention provides pharmaceutical compositions for the treatment of bacterial lung infections, especially, but not limited to, chronic bacterial lung infections and exacerbations of chronic bacterial lung infections in patients with chronic obstructive pulmonary disease (COPD), non-cystic fibrosis bronchiectasis and cystic fibrosis, as well as severe community acquired and hospital-acquired pneumonia, said compositions comprising essentially
1. A pharmaceutical composition comprising
   a) granulocyte-macrophage colony-stimulating factor (GM-CSF) or a fragment or variant thereof, and
   b) fosfomycin in the form of an inorganic or organic salt thereof;
2. A pharmaceutical composition according to 1. above, comprising one or more additional antibiotic or antimicrobial agents;
said compositions being formulated to be suitable for administration via the airways, for example, by the inhalation of an aerosol of a solution of the composition.

The advantage of combining GM-CSF with fosfomycin or fosfomycin in combination with other antibiotics is to combine the effect of GM-CSF to promote the innate defense mechanisms of the lung against bacterial infection with the broad-spectrum bactericidal action of fosfomycin, which has no toxic action on the neutrophils and macrophages through which the healing effect of GM-CSF is exerted. The effect is to achieve a more effective treatment of bacterial lung infections than existing treatments which use antibiotics alone.

In the following detailed description of the invention, details of the scope of the invention and the meaning of the terms used will be given, together with details of the practical performance of the invention.

### Detailed description of the invention

The present invention provides compositions comprising granulocyte-macrophage-colony stimulating factor (GM-CSF) or a fragment or variant thereof, fosfomycin in the form of an inorganic or organic salt thereof, and in a further embodiment, one or more additional antibiotics or antimicrobial agents. Such compositions are useful for the treatment bacterial lung infections, especially chronic bacterial lung infections or exacerbations of the same, and are intended to be administered by via the airways by the inhalation of an aerosol of a solution of the composition or in other ways that will be further described.

### GM-CSF preparations

For practical purposes, the GM-CSF preparations to be used in the present invention will not be purified native human GM-CSF, which could of course be used if it were available in sufficient quantity and problems of possible viral contamination were overcome, but human GM-CSF prepared *in vitro* by recombinant DNA technology. The preparation of human recombinant GM-CSF (hrGM-CSF) in mammalian cells has been described (Wong et al. 1985; Kaushansky et al. 1986). Similar work has led to the production of hrGM-CSF with the non-proprietary name regramostim in Chinese hamster ovarian (CHO) cells (first reported by Moonen et al. 1987). The expression of hrGM-CSF in *Saccharomyces cerevisiae* was reported by Cantrell et al. (1985), leading to the preparation known by the non-proprietary name sargramostim. Sargramostim differs from endogenous human GM-CSF in having a leucine residue instead of a proline residue at position 23 of the pro-peptide and is less glycosylated than either endogenous human GM-CSF or regramostim (Armitage 1998). The expression of hrGM-CSF in *Escherichia coli* was reported by Burgess et al. (1987), leading to the preparation known by the non-proprietary name molgramostim, which is not glycosylated. All three hrGM-CSF preparations, regramostim, sargramostim and molgramostim can be used in the present invention, but only the last two are currently available.

A "functional homologue" of human GM-CSF is herein defined as a polypeptide having at least 50% sequence identity with the known and naturally occurring sequence and sequence variants of human GM-CSF and has one or more functions of the naturally occurring protein. These functions include the following: stimulating the growth and differentiation of hematopoietic precursor cells from various lineages, including granulocytes, macrophages and monocytes, enhancing functional activities of mature effector cells involved in antigen presentation and cell-mediated immunity, including neutrophils, monocytes, macrophages, and dendritic cells. Regramostim, sargramostim and molgramostim may all be said to be functional homologues of naturally occurring human GM-CSF.

Evolutionary conservation between GM-CSF homologues of different closely related species, as assessed by amino-acid sequence alignment, can be used to pinpoint the degree of evolutionary pressure on individual amino-acid residues. Preferably, GM-CSF sequences are compared between species where GM-CSF function is conserved, for example, but not limited to mammals, including rodents, monkeys and apes. Residues under high selective pressure are more likely to represent essential amino acid residues that cannot easily be substituted than residues that change between species. It is evident from the above that a reasonable number of modifications or alterations of the human GM-CSF sequence can be made without interfering with the activity of the GM-CSF molecule according to the invention. Such GM-CSF molecules are herein referred to as functional homologues of human GM-CSF, and may be such variants and fragments of native human GM-CSF as described below.

As used herein, the expression "variant" refers to a polypeptide or protein which is homologous to the index protein, which is naturally occurring human GM-CSF in the present instance, but which differs from the index protein in that one or more amino-acid residues within the sequence of the index protein are substituted by other amino-acid residues. These substitutions may be regarded as "conservative" when an amino-acid residue is replaced by a different amino-acid residue with broadly similar properties, and "non-conservative" when an amino-acid residue is replaced by one of a different type. Broadly speaking, fewer non-conservative substitutions will be possible without altering the biological activity of the polypeptide.

A person skilled in the art will know how to make and assess "conservative" amino-acid substitutions, by which one amino-acid residue is substituted by another having one or more shared chemical and/or physical characteristics. Conservative amino-acid substitutions are less likely to affect the functionality of the protein. Amino acids may be grouped according to their shared characteristics. A conservative amino-acid substitution is a substitution of one amino acid within a predetermined group of amino acids for another amino acid within the same group, within which the amino acids exhibit similar or substantially similar characteristics. Within the meaning of the term "conservative amino acid substitution" as applied herein, one amino acid may be substituted by another within groups of amino acids characterized by having
i) polar side chains (Asp, Glu, Lys, Arg, His, Asn, Gin, Ser, Thr, Tyr and Cys)
ii) non-polar side chains (Gly, Ala, Val, Leu, Ile, Phe, Trp, Pro and Met)
iii) aliphatic side chains (Gly, Ala Val, Leu and Ile)
iv) cyclic side chains (Phe, Tyr, Trp, His and Pro)
v) aromatic side chains (Phe, Tyr and Trp)
vi) acidic side chains (Asp and Glu)
vii) basic side chains (Lys, Arg and His)
viii) amide side chains (Asn and Gin)
ix) hydroxyl side chains (Ser and Thr)
x) sulfur-containing side chains (Cys and Met)
xi) amino acids being monoamino-dicarboxylic acids or monoaminomonocarboxylic-monoamidocarboxylic acids (Asp, Glu, Asn and Gin).

A functional homologue within the scope of the present invention is a polypeptide that exhibits at least 50% sequence identity with a naturally occurring form of human GM-CSF, such as at least 60% sequence identity, for example at least 70% sequence identity, such as at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with a naturally occurring form of human GM-CSF.

Sequence identity can be calculated using a number of well-known algorithms and applying a number of different gap penalties. Any sequence alignment algorithm, such as but not limited to FASTA, BLAST, or GETSEQ, may be used for searching homologues and calculating sequence identity. Moreover, when appropriate, any commonly known substitution matrix, such as but not limited to PAM, BLOSSUM or PSSM matrices, may be applied with the search algorithm. For example, a PSSM (position specific scoring matrix) may be applied via the PSI-BLAST program. Moreover, sequence alignments may be performed using a range of penalties for gap-opening and extension. For example, the BLAST algorithm may be used with a gap-opening penalty in the range 5-12, and a gap-extension penalty in the range 1-2.

Accordingly, a variant or a fragment thereof according to the invention may comprise, within the same variant of the sequence or fragments thereof, or among different variants of the sequence or fragments thereof, at least one substitution, such as a plurality of substitutions introduced independently of one another.

It is clear from the above outline that the same variant or fragment thereof may comprise more than one conservative amino-acid substitution from more than one group of conservative amino acids as defined herein above.

Aside from the twenty standard amino acids and two special amino acids, selenocysteine and pyrrolysine, there are a vast number of "non-standard amino acids" which are not incorporated into protein *in vivo.* Examples of nonstandard amino acids include the sulfur-containing taurine and the neurotransmitters GABA and dopamine. Other examples are lanthionine, 2-aminoisobutyric acid, and dehydroalanine. Further non-standard amino are ornithine and citrulline.

Non-standard amino acids are usually formed through modifications to standard amino acids. For example, taurine can be formed by the decarboxylation of cysteine, while dopamine is synthesized from tyrosine and hydroxyproline is made by a posttranslational modification of proline (common in collagen). Examples of non-natural amino acids are those listed e.g. in 37 C.F.R. section 1.822(b)(4), all of which are incorporated herein by reference.

Both standard and non-standard amino acid residues described herein can be in the "D" or "L" isomeric form.

It is contemplated that a functional equivalent according to the invention may comprise any amino acid including non-standard amino acids. In preferred embodiments, a functional equivalent comprises only standard amino acids.

The standard and/or non-standard amino acids may be linked by peptide bonds or by non-peptide bonds. The term peptide also embraces post-translational modifications introduced by chemical or enzyme-catalyzed reactions, as are known in the art. Such post-translational modifications can be introduced prior to partitioning, if desired. Amino acids as specified herein will preferentially be in the L-stereoisomeric form. Amino acid analogs can be employed instead of the 20 naturally occurring amino acids. Several such analogs are known, including fluorophenylalanine, norleucine, azetidine-2-carboxylic acid, S-aminoethyl cysteine, 4-methyl tryptophan and the like.

In one embodiment of the present invention, the GM-CSF variant comprises a conjugate capable of prolonging half-life of the active ingredient, such as for example albumin or a fatty acid.

Suitable variants will be at least 60% identical, preferably at least 70%, and accordingly, variants preferably have at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with the predetermined sequence of a naturally occurring form of human GM-CSF.

Functional homologues may further comprise chemical modifications such as ubiquitination, labeling (e.g., with radionuclides, various enzymes, etc.), pegylation (derivatization with polyethylene glycol), or by insertion (or substitution by chemical synthesis) of amino acids such as ornithine, which do not normally occur in human proteins.

In addition to the peptidyl compounds described herein, sterically similar compounds may be formulated to mimic the key portions of the peptide structure and such compounds may also be used in the same manner as the polypeptides of the invention. This may be achieved by techniques of modelling and chemical designing known to those of skill in the art. For example, esterification and other alkylations may be employed to modify the amino terminus (N-terminus) of, e.g., a di-arginine peptide backbone, to mimic a tetrapeptide structure. It will be understood that all such sterically similar constructs fall within the scope of the present invention.

Peptides with N-terminal alkylations and C-terminal esterifications are also encompassed by the present invention. Functional equivalents also comprise glycosylated and covalent or aggregative conjugates formed with the same molecules, including dimers or unrelated chemical moieties. Such functional equivalents are prepared by linkage of functionalities to groups which are found in a fragment that includes any one or both of the N- and C-termini, by means known in the art.

The term "fragment thereof may refer to any portion of the given amino-acid sequence. Fragments may comprise more than one portion from within the full-length protein, joined together. Suitable fragments may be deletion or addition mutants. The addition of at least one amino acid may be an addition of from preferably 2 to 250 amino acids, such as from 10 to 20 amino acids, for example from 20 to 30 amino acids, such as from 40 to 50 amino acids. Fragments may include small regions from the protein or combinations of these. The deletion and/or the addition may, independently of one another, be a deletion and/or an addition within a sequence and/or at the end of a sequence.

Deletion mutants suitably comprise at least 20 or 40 consecutive amino acid and more preferably at least 80 or 100 consecutive amino acids in length. Accordingly, such a fragment may be a shorter sequence taken from the sequence of human GM-CSF comprising at least 20 consecutive amino acids, for example at least 30 consecutive amino acids, such as at least 40 consecutive amino acids, for example at least 50 consecutive amino acids, such as at least 60 consecutive amino acids, for example at least 70 consecutive amino acids, such as at least 80 consecutive amino acids, for example at least 90 consecutive amino acids, such as at least 95 consecutive amino acids, such as at least 100 consecutive amino acids, such as at least 105 amino acids, for example at least 110 consecutive amino acids, such as at least 115 consecutive amino acids, for example at least 120 consecutive amino acids, wherein said deletion mutants preferably has at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with a naturally occurring form of human GM-CSF.

It is preferred that functional homologues of GM-CSF comprise at most 500, more preferably at most 400, even more preferably at most 300, yet more preferably at most 200, such as at most 175, for example at most 160, such as at most 150 amino acids, for example at most 144 amino acids.

There are two known variants of human GM-CSF: a T1151 substitution in variant 1 and an 1117T substitution in variant 2. Accordingly, in one embodiment of the invention, a functional homologue of GM-CSF comprises a sequence with high sequence identity to human GM-CSF NO: 1 or any of the splice variants.

Analogues of GM-CSF are, for example, described in U.S. Pat. Nos. 5,229,496, 5,393,870, and 5,391,485. Such analogues are also functional equivalents comprised within the present invention.

In one embodiment, GM-CSF is used according to the present invention in homo- or heteromeric form. Homo- and heteromeric forms of GM-CSF may comprise one or more GM-CSF monomers or functional homologous of GM-CSF as defined herein above. Homo- and heteromers include dimers, trimers, tetramers, pentamers, hexamers, heptamers, octamers, nonamers and decamers.

In one embodiment, a homodimer, trimer or tetramer of GM-CSF is used.

The amino-acid sequence of the precursor (including the signal peptide) form of GM-CSF of Homo sapiens (SEQ ID NO:1) is:

MWLQSLLLLG TVACSISAPA RSPSPSTQPW EHVNAIQEAR RLLNLSRDTA
AEMNETVEVI SEMFDLQEPT CLQTRLELYK QGLRGSLTKL KGPLTMMASH

YKQHCPPTPE TSCATQIITF ESFKENLKDF LLVIPFDCWE PVQE

The amino-acid sequence of the corresponding mature protein (SEQ ID NO: 2) is:

Functional homologues of a naturally occurring form of human GM-CSF according to the present invention may be commercially available, e.g. sargramostim (Leukine®; Immunex, Seattle, WA, USA).

### Recombinant production of GM-CSF

GM-CSF or functional variants or homologues thereof can be produced in various ways, such as isolation from for example human or animal serum or from expression in cells, such as prokaryotic cells, yeast cells, insect cells, mammalian cells or in cell-free systems.

In one embodiment of the invention, GM-CSF is produced recombinantly by host cells. Thus, in one aspect of the present invention, GM-CSF is produced by host cells comprising a first nucleic acid sequence encoding the GM-CSF operably associated with a second nucleic acid sequence capable of directing expression in said host cells. The second nucleic acid sequence may thus comprise or even consist of a promoter that will direct the expression of protein of interest in said cells. A skilled person will be readily capable of identifying useful second nucleic acid sequence for use in a given host cell.

The process of producing a recombinant GM-CSF in general comprises the steps of
- providing a host cell
- preparing a gene expression construct comprising a first nucleic acid sequence encoding the GM-CSF operably linked to a second nucleic acid sequence capable of directing the expression of said protein of interest in the host cell
- transforming the host cell with the construct
- cultivating the host cell, thereby obtaining expression of the GM-CSF.

The recombinant GM-CSF thus produced may be isolated by any conventional method, such as any of the methods for protein isolation described herein below. The skilled person will be able to identify suitable protein isolation steps for purifying the GM-CSF.

In one embodiment of the invention, the recombinantly produced GM-CSF is excreted by the host cells. When the GM-CSF is excreted, the process of producing a recombinant protein of interest may comprise the steps of
- providing a host cell
- preparing a gene expression construct comprising a first nucleic acid sequence encoding the GM-CSF operably linked to a second nucleic acid sequence capable of directing the expression of said protein of interest in said host cell
- transforming said host cell with the construct
- cultivating the host cell, thereby obtaining expression of the GM-CSF and secretion of the GM-CSF into the culture medium
- thereby obtaining culture medium containing the GM-CSF.

The composition comprising GM-CSF and nucleic acids may thus in this embodiment of the invention be the culture medium or a composition prepared from the culture medium.

In another embodiment of the invention, said composition is an extract prepared from animals, parts thereof or cells or an isolated fraction of such an extract.

In an embodiment of the invention, the GM-CSF is recombinantly produced *in vitro* in host cells and isolated from cell lysate, cell extract or from tissue culture supernatant. In a more preferred embodiment, the GM-CSF is produced by host cells that are modified in such a way that they express the relevant GM-CSF. In an even more preferred embodiment of the invention, said host cells are transformed to produce and excrete the relevant GM-CSF.

Pharmaceutical compositions according to the present invention may comprise GM-CSF or functional variants or homologues thereof, such that a solution of the composition for airways administration has a concentration in the range of 25 µg/mL to 1000 µg/mL, such as in the range of 75 µg/mL to 500 µg/mL, or such as in the range of 125 µg/mL to 400 µg/mL.

### Fosfomycin preparations

The fosfomycin preparations that fall within the scope of the present invention are compounds and salts of compounds that comprise the structure (-)(IR, 2S)-1,2-epoxypropylphosphonic acid, systematic name [(2R,3S)-3-methyloxiran-2-yl]phosphonic acid, formula weight 138.1 Da. Although the free acid form of this structure can be obtained and is used as a basis for calculating the effective amounts and concentrations of fosfomycin, the free acid is unstable as well as highly irritating and the antibiotic is presented for clinical use as inorganic or organic salts. Non-limiting examples of inorganic counter-ions of fosfomycin salts within the scope of the present invention are sodium, calcium, potassium, lithium, ammonium, magnesium. Non-limiting examples of organic counter-ions of fosfomycin salts within the scope of the present invention are trometamol (also known as tromethamine or tris, systematic name 2-amino-2-hydroxymethyl-propane-1,3-diol), phenylethylamine, and a large number of other biocompatible organic amines. The principal forms of fosfomycin in current use that come within the scope of this invention are:
i) Fosfomycin disodium, formula weight 182.0 Da, pH of 5% solution 9.0-10.5. This salt is highly soluble in water, irritant to the stomach, and is principally used for intravenous injection.
ii) Fosfomycin calcium monohydrate, formula weight 194.1 Da, pH of 0.4% solution 8.1-9.6. This salt is sparingly soluble in water but is less irritating to the stomach and is used for oral treatment. Its bioavailability may be as low as 12% (Bergan 1990).
iii) Fosfomycin trometamol, formula weight 259.2 Da, pH of 5% solution 3.5-5.5. This salt is highly soluble in water, is well tolerated when given orally and is used especially as a single-dose oral treatment for lower urinary tract infection, showing a bioavailability of about 40%.

Common to these preparations is that they are stable for at least 3 years as dry powders at 25°C, but show a pH-dependent instability in aqueous solution, at 25°C losing 10% of fosfomycin activity within 10 h at pH 3, 2 months at pH 6.5 and 2 years at pH 9.75. Reduced stability at neutral (and low) pH must be taken into in account when formulating compositions for airways administration with a suitable shelf life.

### Combination with other antimicrobial agents

A composition of the present invention may also contain one or more additional antimicrobial agents to potentiate its bactericidal action on important pathogens such as *Staphylococcus aureus, Pseudomonas aeruginosa* or *Moraxella catarrhalis,* prevent the overgrowth of any strains that develop resistance on prolonged treatment, or broaden the antimicrobial spectrum to include non-bacterial pathogens, including but not limited to fungi. Non-limiting examples of antimicrobial agents that may be included in the composition are: penicillins, cephalosporins, aminoglycosides, macrolides, vancomycin, lincomycin, clindamycin, fluoroquinolones, metronidazole, clotrimazole, ketoconazole and nystatin. Particularly suitable for inclusion are agents which show a synergic bactericidal action with fosfomycin, non-limiting examples of which are: penicillin, ampicillin, carbenicillin, methicillin, oxacillin, mezlocillin, piperacillin, aztreonam, imipenem, cephalexin, cephalothin, cefamandole, cefoxitin, cefmetazole, cefotaxime, cefazolin, cefoperazone, cefsulodine, ceftadizime, cefepime, streptomycin, gentamicin, kanamycin, netilmicin, tobramycin, amikacin, erythromycin, midecamycin, vancomycin, lincomycin, clindamaycin, teicoplanin, daptomycin, ciprofloxacin, ofloxazine, levofloxazin, pefloxacin, sparfloxacin. Certain antibiotics are incompatible with fosfomycin in aqueous solution, such as ampicillin, methampicillin, cephaloridine, cephalothin, streptomycin, gentamicin, kanamycin, a feature that makes them unsuitable for compositions in aqueous solution within the scope of this invention.

In one embodiment, the additional antibiotic or antibacterial agent is selected from the list of amikacin, aztreonam, cefotaxime, ceftaroline, ceftriaxone, clindamycin, colistin, flucloxacillin, meropenem, metronidazole, mezlocillin, polymyxin B or vancomycin.

In another embodiment, the additional antibiotic is at least a combination of aztreonam and amikacin.

As all the above compounds are known to the skilled person, it will not present an undue burden to the skilled person to choose from the list of compounds which compound(s) to combine with the GM-CSF and fosfomycin treatment on the basis of test data on the identity of the infective organisms that are to be treated.

### Dose

By "effective amount" of the pharmaceutical compositions of the present invention is meant a dose, which, when administered to a subject in need thereof, achieves a concentration which has a beneficial biological effect, i.e. by eliminating or alleviating the symptoms and signs of lung infection. Such an effective amount may be determined by a patient's attending physician and is readily ascertained by one of ordinary skill in the art.

The effective amounts and dosages of the ingredients of the composition are determined in relation to body weight or body surface area as for systemic treatments, though the relationship of lung surface area to be treated by airways administration to body weight or body surface area will vary between individual patients. It is evident that the total dosages given by airways administration should not exceed the maximum tolerated doses that would be given by conventional parenteral treatment, and would in most cases be considerably lower than the doses used for said treatment.

The effective amount of GM-CSF or a functional variant or homologue thereof, for airways administration, may be from 3 microgram (µg) to 300 µg per kilogram of body weight per day, such as in the range of 5 µg to 150 µg per kilogram per day, and especially in the range of 5 µg to 50 µg per kilogram per day. The daily dose may be divided into two or more equal fractional doses per day. The effective amount is expressed in terms of the amount to be given of a fully functional homologue of human GM-CSF such as molgramostim and is adjusted according to functional activity of the homologue used.

In one embodiment, GM-CSF is administered in a dose within the range of 25 µg to 1000 µg of GM-CSF per dose, such as from 50 µg to 800 µg per dose, such as from 50 µg to 600 µg per dose, such as from 50 µg to 400 µg per dose. In a preferred embodiment, GM-CSF is administered in a dose within the range of 50 µg to 250 µg per dose between 1 and 6 times/day, such as once daily, 2 times daily, 3 times daily, 4 times daily, 5 times daily or 6 times daily.

In practical terms, a pharmaceutical composition of the present invention comprises GM-CSF or a fragment or variant thereof, such that a solution of it for airways administration has a concentration in the range of 50 µg/mL to 1000 µg/mL, such as in the range of 75 µg/mL to 500 µg/mL, or such as in the range of 75 µg/mL to 300 µg/mL.

The effective amount of a suitable salt of fosfomycin for airways administration may be from 0.7 mg to 20 mg per kilogram of body weight per day, such as in the range of 1 mg to 10 mg per kilogram of body weight per day, and especially in the range of 2 mg to 5 mg per kilogram of body weight per day. The effective amount is expressed in terms of the content of fosfomycin free acid in the preparation used.

In practical terms, a pharmaceutical composition of the present invention comprises a fosfomycin salt dissolved in an aqueous medium to achieve a concentration in the range of 10 mg/mL to 50 mg/mL such as in the range of 20 mg/mL to 40 mg/mL in terms of fosfomycin free acid.

In a preferred embodiment, fosfomycin is administered in a dose within the range of 20 mg to 1 g per dose, such as from 20 mg to 500 mg per dose, such as from 40 mg to 400 mg, such as from 80 mg per dose to 300 mg per dose, such as from 120 mg to 250 mg per dose.

In a highly preferred embodiment, fosfomycin is administered in a dose within the range from 120 mg to 500 mg per dose.

The effective amount of an additional antimicrobial agent for topical application is known in the art and may be added to the pharmaceutical compositions of the present invention in an amount that is adjusted so that when an effective amount of GM-CSF and fosfomycin is given, an effective amount of the additional antimicrobial agent is also given. In practice, this means that fusidic acid, for example, will be added to the composition in a similar amount to that of fosfomycin, while tobramycin, for example, will be added in an amount that is between 1% and 10% of the amount of fosfomycin.

The effective daily dose is preferably administered in divided doses twice a day, but may be given once a day, in divided doses, two times a day, three times a day, four times a day, five times a day, six times a day, seven times a day, eight times a day, nine times a day, ten times a day, eleven times a day, twelve times a day, thirteen times a day, fourteen times a day or fifteen times a day. In a preferred embodiment, the effective daily dose is administered in divided doses two times a day, three times a day, four times a day, five times a day, or six times a day.

Duration of dosing will typically range from 1 day to about 1 month, such as in the range of 1 day to 2 days, 2 days to 3 days, 3 days to 4 days, 4 days to 5 days, 5 days to 6 days, or in the range of 1 week to 2 weeks, 2 weeks to 3 weeks, 3 weeks to 4 weeks.

The transformation of a resting macrophage into a fully immunocompetent dendritic cell after *in vitro* incubation of macrophages with GM-CSF takes approximately 10 days. In one embodiment, a duration of a dose has the length allowing for said a transformation, thus the duration can be in the range of 7 days to 14 days, such as 8 days to 12 days, for example 8 days, or 9 days, or 10 days, or 11 days, or 12 days.

A dose regime may alternate between periods of administration of the pharmaceutical composition according to the present invention and periods with no administration (a pause in treatment). A period with a pause of treatment in such a dose regime may last for 5 days to 10 days, for example 5 days, or 6 days, or 7 days, or 8 days, or 9 days, or for example 10 days or more, for example 1 to 4 months, all at the discretion of the attending physician.

In one embodiment, a dosage unit according to the present invention comprises 150 microgram GM-CSF and 40 mg to 400 mg fosfomycin for administration 2-6 times daily, such as 3 times daily.

### Formulations

The pharmaceutical composition of the present invention may be in the form of a powder, emulsion, suspension, or solution.

A preferred formulation to supply the pharmaceutical composition as a dry powder in a capped vial, to be reconstituted as a solution for nebulization by adding a given volume of sterile water from a second vial, to be used within one hour. The water for dissolving the formulation may contain the empirically determined amount of acid or base with buffering ions to produce a solution of the composition that has a near-neutral pH, or more preferably, the powder in the capped vial with the active pharmaceutical ingredients contains solid, physiologically acceptable organic acid in an amount empirically adjusted to give a near-neutral pH on dissolving in the given volume of water. By near-neutral pH is meant a pH between 6 and 8, preferably 7. Examples of acids that may be used include, but are not limited to, succinic acid and citric acid. The amounts of fosfomycin salt and the other solutes of the reconstituted solution are adjusted to yield a solution that has an osmolarity as close as possible to and no more than twice the osmolarity of plasma, to avoid the provocation of cough and bronchoconstriction on inhalation.

In one embodiment, the formulations of the present invention is for administration by inhalation of spray by use of a spacer in position between the MDI and the patient.

**Example 2** gives the composition of a formulation to provide an effective dose of GM-CSF and fosfomycin as a twice-daily inhalation.

**Example 3** gives the composition of formulation to provide an effective dose of GM-CSF and fosfomycin together with meropenem as a twice-daily inhalation to provide a strong action against *Pseudomonas aeruginosa* and *Prevotella.*

Solutes to be added to the water for dissolving the pharmaceutical composition will include hydrochloric acid and biocompatible buffering agents, non-limiting examples being sodium dihydrogen phosphate and disodium hydrogen phosphate, sodium carbonate and bicarbonate, and tromethamine. Tonicity-adjusting agents, such as for example sodium chloride or calcium chloride, may also be added.

Formulations according to the present invention may comprise pharmaceutically acceptable carriers and excipients including microspheres, liposomes, micelles, microcapsules, nanoparticles or the like. Both the GM-CSF and the antibiotic components of the pharmaceutical composition may, for example, be formulated in liposomes with an outer fatty layer and a core of water phase in which the component is dissolved. The lipid layer of such formulations aids the penetration and effectiveness of the components against bacterial biofilms (Marier et al. 2003; Meers et al. 2008).

Conventional liposomes are typically composed of phospholipids (neutral or negatively charged) and/or cholesterol. The liposomes are vesicular structures based on lipid bilayers surrounding aqueous compartments. They can vary in their physicochemical properties such as size, lipid composition, surface charge and number and fluidity of the phospholipids bilayers. The most frequently used lipid for liposome formation are: 1,2-dilauroyl-*sn*-glycero-3-phosphocholine (DLPC), 1,2-dimyristoyl-*sn*-glycero-3-phosphocholine (DMPC), 1,2-dipalmitoyl-*sn*-glycero-3-phosphocholine (DPPC), 1,2-distearoyl-*sn*-glycero-3-phosphocholine (DSPC), 1,2-dioleoyl-*sn*-glycero-3-phosphocholine (DOPC), 1,2-dimyristoyl-*sn*-glycero-3-phosphoethanolamine (DMPE), 1,2-dipalmitoyl-*sn-*glycero-3-phosphoethanolamine (DPPE), 1,2-dioleoyl-*sn*-glycero-3-phosphoethanolamine (DOPE), 1,2-dimyristoyl-*sn*-glycero-3-phosphate (monosodium salt) (DMPA), 1,2-dipalmitoyl-*sn*-glycero-3-phosphate (monosodium salt) (DPPA), 1,2-dioleoyl-*sn*-glycero-3-phosphate (monosodium salt) (DOPA), 1,2-dimyristoyl-*sn*-glycero-3-[phospho-rac-(1-glycerol)] (sodium salt) (DMPG), 1,2-dipalmitoyl-*sn*-glycero-3-[phospho-rac-(1-glycerol)] (sodium salt) (DPPG), 1,2-dioleoyl-*sn*-glycero-3-[phospho-rac-(1-glycerol)] (sodium salt) (DOPG), 1,2-dimyristoyl-*sn*-glycero-3-[phospho-l-serine] (sodium salt) (DMPS), 1,2-dipalmitoyl-*sn*-glycero-3-[phospho-l-serine) (sodium salt) (DPPS), 1,2-dioleoyl-*sn*-glycero-3-[phospho-l-serine] (sodium salt) (DOPS), 1,2-dioleoyl-*sn-*glycero-3-phosphoethanolamine-n-(glutaryl) (sodium salt) and 1,1',2,2'-tetramyristoyl cardiolipin (ammonium salt). Formulations composed of DPPC in combination with other lipids or modifiers of liposomes are preferred, e.g. in combination with cholesterol and/or phosphatidylcholine.

A useful way of producing liposomes is to attach hydrophilic polymer polyethylene glycol (PEG) covalently to the outer surface of the liposome. Some of the preferred lipids are: 1,2-dipalmitoyl-*sn*-glycero-3-phosphoethanolamine-n-[methoxy(polyethylene glycol)-2000] (ammonium salt), 1,2-dipalmitoyl-*sn-*glycero-3-phosphoethanolamine-n-[methoxy(polyethylene glycol)-5000] (ammonium salt), 1,2-dioleoyl-3-trimethylammonium-propane (chloride salt) (DOTAP).

Possible lipids applicable for liposomes are supplied by e.g. Avanti, Polar Lipids, Inc., Alabaster, AL, USA. Additionally, the liposome suspension may include lipid-protective agents which protect lipids against free-radical and lipid-peroxidative damage on storage. Lipophilic free-radical quenchers, such as alpha-tocopherol and water-soluble iron-specific chelators, such as ferrioxamine, are preferred.

Several methods are available for preparing liposomes, as described in, e.g., Szoka et al. (1980), U.S. Pat. Nos. 4, 235,871, 4,501,728 and 4,837,028, all of which are incorporated herein by reference. Another method produces multi-lamellar vesicles of heterogeneous sizes. In this method, the vesicle-forming lipids are dissolved in a suitable organic solvent or solvent system and dried under vacuum or an inert gas to form a thin lipid film. If desired, the film may be re-dissolved in a suitable solvent, such as tertiary butanol, and then lyophilized to form a more homogeneous lipid mixture which is in a more easily hydrated powder-like form. This film is covered with an aqueous solution of the targeted drug and the targeting component and allowed to hydrate, typically over a 15-60 minute period with agitation. The size distribution of the resulting multi-lamellar vesicles can be shifted toward smaller sizes by hydrating the lipids under more vigorous agitation conditions or by adding solubilizing detergents such as deoxycholate.

### Administration

Administration of an effective amount of the pharmaceutical composition is by airways administration, such as by inhalation of an aerosol of a solution of the composition or of the composition in fine powder form, or by intratracheal, intrabronchial or bronchoalveolar administration.

Methods of intratracheal, intrabronchial or bronchoalveolar administration include, but are not limited to, spraying, lavage, inhalation, flushing or installation, using as fluid a physiologically acceptable composition in which the pharmaceutical composition has been dissolved. When used herein the terms "intratracheal, intrabronchial or intraalveolar administration" include all forms of such administration whereby the composition is applied into the trachea, the bronchi or the alveoli, whether by the instillation of a solution of the composition, by applying the composition in a powder form, or by allowing the composition to reach the relevant part of the airway by inhalation of the composition as an aerosolized or nebulized solution or suspension or inhaled powder, with or without added stabilizers or other excipients.

Methods of intrabronchial or intraalveolar administration include, but are not limited to, bronchoalveolar lavage (BAL) according to methods well known to those skilled in the art, using as a lavage fluid a physiologically acceptable composition in which the composition has been dissolved, or indeed by any other effective form of intrabronchial administration including the use of inhaled powders containing the composition in dry form, with or without excipients, or by the direct application of the composition, in solution or suspension or powder form during bronchoscopy. Methods for intratracheal administration include, but are not limited to, blind tracheal washing with a similar solution of dissolved composition or with a suspension of the composition, or the inhalation of nebulized fluid droplets containing the dissolved composition or a suspension of the composition, obtained by use of any nebulizing apparatus adequate for this purpose.

In another embodiment, intratracheal, intrabronchial or intraalveolar administration of the composition does not include inhalation of the product, but the instillation or application of a solution of the composition or a powder or a gel containing the composition into the trachea or lower airways.

Other preferred methods of administration may include using the following devices:
1. Pressurized nebulizers using compressed air/oxygen mixture
2. Ultrasonic nebulizers
3. Electronic micropump nebulizers (e.g. Aeroneb Professional Nebulizer)
4. Metered dose inhaler (MDI)
5. Dry powder inhaler systems (DPI).
The aerosol may be delivered by a) facemasks or b) endotracheal tubes in intubated patients during mechanical ventilation (device 1, 2 and 3). The devices 4 and 5 can also be used by the patient without assistance, provided that the patient is able to self-activate the aerosol device.

Improved penetration of the inhaled composition to its target site, which includes the small airways (bronchioles and alveoli), may be obtained by:
(i) Giving a higher dose-rate of the inhaled composition in order to achieve the wanted effect.
(ii) Applying continuous positive airway pressure (CPAP) with spontaneous breathing or extrinsic positive end-expiratory pressure (PEEP) in mechanical ventilation in order to facilitate the delivery of the inhaled preparation to the distal airways and enhance its effect.

The two applications of increased airway pressure (CPAP and PEEP) may increase the collateral ventilation (CV) via the ventilation pores between the terminal units of peripheral airways. The phenomenon of CV can be particularly useful in pulmonary disease with anatomical partial or total block of the airways, since it can increase delivery of drugs to the site of interest, which is the peripheral airways. By exploiting the occurrence of CV, CPAP and PEEP may cause air to bypass obstructed airways through collateral channels including interalveolar pores, bronchiole-alveolar communications, and interbronchiolar pathways. Resistance through these channels located at the small airways increases with decreasing lung volume. Functional blockage of the airways to the passage of an aerosol may thus be alleviated by exploiting the occurrence of CV, which facilitates the distribution of the compound into the airways beyond the level of obstruction. Thus, in one embodiment of the present invention, the compound is administered by inhalation combined with collateral ventilation, such as CPAP and/or PEEP.

### Embodiments

1. A pharmaceutical composition comprising
   a. granulocyte-macrophage colony-stimulating factor (GM-CSF) or a fragment or variant thereof, and
   b. fosfomycin or an inorganic or organic salt thereof
2. The pharmaceutical composition according to embodiment 1 comprising one or more additional antibiotic or antimicrobial agents.
3. The composition according to embodiment 2, wherein the one or more additional antibiotic or antimicrobial agent is selected from the list of penicillins, cephalosporins, aminoglycosides, macrolides, vancomycin, lincomycin, clindamycin, fluoroquinolones, metronidazole, clotrimazole, ketoconazole and nystatin.
4. The composition according to embodiment 2 or 3, wherein the one or more additional antibiotic or antimicrobial agent is selected from the list of amikacin, aztreonam, cefotaxime, ceftaroline, ceftriaxone, clindamycin, colistin, flucloxacillin, meropenem, metronidazole, mezlocillin, polymyxin B or vancomycin.
5. The composition according to any one of the previous embodiments, wherein the lesion is infected with bacteria from any one of the genera *Staphylococcus, Streptococcus, Neisseria, Escherichia, Serratia, Salmonella, Shigella, Pseudomonas, Haemophilus, Vibrio,* and *Klebsiella pneumoniae, Proteus vulgaris, Proteus mirabilis, Moraxella catarrhalis, Burkholderia cepacia, Mycobacterium spp., Stenotrophomonas maltophilia, Veillonella, Fusobacterium* and *Prevotella.*
6. The pharmaceutical composition according to any one of the previous embodiments, wherein the composition is for use in an individual suffering from a bacterial lung infection.
7. The pharmaceutical composition according to any one of the previous embodiments, wherein the composition is for use in an individual suffering from a bacterial lung infection which occurs concurrently with a condition which compromises the natural host defense mechanisms in the airways.
8. The pharmaceutical composition according to any one of the previous embodiments, wherein the composition is for use in an individual suffering from a bacterial lung infection which occurs concurrently with a condition which compromises the natural host defense mechanisms in the airways, wherein the condition which compromises the natural host defense of the airways is the occurrence of severe illness, including severe systemic infections, trauma, whether non-surgical or surgical in origin, major inflammatory diseases, and indeed any of a very large number of diseases known to adversely affect innate and/or acquired immunity in general, respiratory complications of severe illness and shock, such as acute respiratory distress syndrome, iatrogenic causes of reduced host defense mechanisms in the lungs, such as immunosuppressive or cytotoxic treatments.
9. The pharmaceutical composition according to any one of embodiments 1 to 4 wherein the GM-CSF and/or the antibiotics are in liposomal or micelle or microcapsule or nanoparticle formulations.
10. The pharmaceutical composition according to any one of the previous embodiments, wherein the composition is a powder or an aerosol for inhalation.
11. The pharmaceutical composition according to any one of the preceding embodiments wherein the GM-CSF variant is at least 70% identical to SEQ ID NO: 1 or 2.
12. The pharmaceutical composition according to any one of the preceding embodiments wherein the GM-CSF fragment comprises at least 50 contiguous amino acid residues of any one of SEQ ID NO: 1 or 2.
13. The pharmaceutical composition according to embodiment 8, wherein the fragment is at least 70% identical to SEQ ID NO: 1 or 2 in the range of overlap.
14. The pharmaceutical composition according to any one of the preceding embodiments, wherein the fosfomycin salt is fosfomycin disodium or fosfomycin calcium or fosfomycin trometamol, also known as fosfomycin tromethamine.
15. The pharmaceutical composition according to any one of the preceding embodiments, further comprising one or more additional antibiotic or antimicrobial agents.
16. The pharmaceutical composition according to any one of the preceding embodiments, wherein the GM-CSF variant comprises a conjugate capable of prolonging the half-life of the GM-CSF.
17. The pharmaceutical composition according to the preceding embodiment, wherein the conjugate capable of prolonging half-life of the GM-CSF variant is albumin or a fatty acid.
18. The pharmaceutical composition according to any one of the preceding embodiments, wherein the composition is for use in a subject suffering from acute or chronic lung infection.
19. The pharmaceutical composition according to any one of the preceding embodiments, wherein the composition is for use in a subject suffering from any one of hospital-acquired pneumonia, severe community-acquired pneumonia, ventilator-associated pneumonia or pneumonia in intensive care unit.
20.The pharmaceutical composition according to any one of the preceding embodiments, wherein at least 200 micrograms, or at least 250 micrograms or at least 300 micrograms of GM-CSF is administered per day.
21. The pharmaceutical composition according to any one of the preceding embodiments, wherein the composition is made for administration of GM-CSF in an amount of from 25 microgram to 750 microgram per dose.
22.The pharmaceutical composition according to any one of the preceding embodiments, wherein the composition is made for administration of from 20 mg to 1 g of fosfomycin per dose, such as from 120 mg to 500 mg of fosfomycin per dose, such as from 250 mg to 500 mg of fosfomycin per dose.
23.The pharmaceutical composition according to any one of the preceding embodiments, wherein the composition is made for administration of a dosage comprising from 25 microgram to 750 microgram of GM-CSF and from 20 mg to 1 g of fosfomycin, such as from 120 mg to 500 mg of fosfomycin, such as from 250 mg to 500 mg of fosfomycin per dose.
24.The pharmaceutical composition according to any one of the preceding embodiments, wherein fosfomycin is administered at a daily dose ranging from 0.7 mg to 20 mg per kilogram of body weight, such as in the range of 1 mg to 10 mg per kilogram of body weight, and especially in the range of 2 mg to 5 mg per kilogram of body weight.
25. The pharmaceutical composition according any one of the preceding embodiments, wherein the fosfomycin salt is fosfomycin disodium or fosfomycin calcium or fosfomycin trometamol, also known as fosfomycin tromethamine.
26.A pharmaceutical composition comprising: a. granulocyte-macrophage colony-stimulating factor (GM-CSF) in the form of molgramostim or sargramostim or regramostim, and b. fosfomycin in the form of an inorganic or organic salt thereof for use in the treatment or alleviation of bacterial lung infections or colonization, wherein the composition is formulated for administration via the airways by inhalation or by intratracheal intrabronchial or intraalveolar administration.
27. The pharmaceutical composition for use in the treatment or alleviation according to embodiment 26, wherein the composition is for inhalation as a powder or as an aerosol.
28. The pharmaceutical composition for use in the treatment or alleviation according to embodiments 26 and 27, wherein the GM-CSF and/or the fosfomycin salt and any additional antibiotic or antimicrobial agent are formulated in separate liposomal or micelle or microcapsule or nanoparticle populations and mixed before use.
29. The pharmaceutical composition for use in the treatment or alleviation according to any one of embodiments 26-28, wherein the GM-CSF and/or the fosfomycin salt and or any additional antibiotic or antimicrobial agent are in separate formulations made for concomitant use.
30. The pharmaceutical composition for use in the treatment or alleviation according to any one of the embodiments 26-29, comprising GM-CSF at a concentration of 50 micrograms per milliliter to 1000 micrograms per milliliter.
31. The pharmaceutical composition for use in the treatment or alleviation according to any one of the embodiments 26-30, wherein at least 200 micrograms, or at least 250 micrograms or at least 300 micrograms of GM-CSF is administered per day.
32. The pharmaceutical composition for use in the treatment or alleviation according to any one of the embodiments 26-31, wherein the composition is formulated for administration of GM-CSF in an amount of from 25 micrograms to 750 micrograms per dose.
33. The pharmaceutical composition for use in the treatment or alleviation according to any one of the embodiments 26-32, wherein the composition is made for administration of from 20 milligrams to 1 gram of fosfomycin per dose, such as from 120 milligrams to 500 milligrams of fosfomycin per dose.
34. The pharmaceutical composition for use in the treatment or alleviation according to any one of the embodiments 26-33, wherein the composition is made for administration of a dosage comprising from 25 micrograms to 750 micrograms of GM-CSF and from 20 milligrams to 1 gram of fosfomycin, such as from 120 milligrams to 500 milligrams of fosfomycin.

### Examples

The following non-limiting examples further illustrate the present invention.

### Example 1: Sequences

SEQ ID NO: 1 – Human GM-CSF precursor
>sp|P04141|CSF2_HUMAN Granulocyte-macrophage colony-stimulating factor
OS=Homo sapiens
MWLQSLLLLGTVACSISAPARSPSPSTQPWEHVNAIQEARRLLNLSRDTAAEM
NETVEVISEMFDLQEPTCLQTRLELYKQGLRGSLTKLKGPLTMMASHYKQHC
PPTPETSCATQIITFESFKENLKDFLLVIPFDCWEPVQE

SEQ ID NO: 2 – mature human GM-CSF
>sp|P04141|18-144




### Example 2: Composition of a formulation to provide an effective dose of GM-CSF and fosfomycin as a twice-daily inhalation.

Human recombinant GM-CSF (as molgramostim or sargramostim) 150 µg; fosfomycin disodium 53 mg (approximately equivalent to 40 mg of fosfomycin free acid), succinic acid 20-40 mg (amount to be determined to give a final pH of 7 when the mixture is dissolved), water for injection 2 mL. The dry solutes are packed in a capped, evacuated vial, and another capped vial contains the water for injection.

### Example 3: Composition of formulation to provide an effective dose of GM-CSF and fosfomycin together with meropenem as a twice-daily inhalation to provide a strong action against Pseudomonas aeruginosa and Prevotella.

Human recombinant GM-CSF (as molgramostim or sargramostim) 150 µg; fosfomycin disodium 53 mg (approximately equivalent to 40 mg of fosfomycin free acid), meropenem 200 mg, succinic acid 20-40 mg (amount to be determined to give a final pH of 7 when the mixture is dissolved), water for injection 2 mL. The dry solutes are packed in a capped, evacuated vial, and another capped vial contains the water for injection.

### References

Armitage JO (1998) Emerging applications of recombinant human granulocyte-macrophage colony-stimulating factor. Blood 92:4491-4508.
Baleeiro CE, Christensen PJ, Morris SB, et al. (2006) GM-CSF and the impaired pulmonary innate immune response following hyperoxic stress. Am J Physiol Lung Cell Mol Physiol 291:L1246-L1255.
Beasley V, Joshi PV, Singanayagam A (2012) Lung microbiology and exacerbations in COPD. Int J COPD 7:555-569.
Bergan T (1990) Degree of absorption, pharmacokinetics of fosfomycin trometamol and duration of urinary antibacterial activity. Infection 18 Suppl 2:S65-S69.
Brown CB, Pihl CE, Kaushansky K (1994) Mapping of human granulocyte-macrophage-colony-stimulating-factor domains interacting with the human granulocyte-macrophage-colony-stimulating-factor-receptor alpha-subunit. Eur J Biochem 225:873-880.
Brown ED, Vivas EI, Walsh CT, Kolter R (1995) MurA (MurZ), the enzyme that catalyzes the first committed step in peptidoglycan biosynthesis, is essential in Escherichia coli. J Bacteriol 177:4194-4197.
Burgess AW, Begley CG, Johnson GR, et al. (1987) Purification and properties of bacterially synthesized human granulocyte-macrophage colony stimulating factor. Blood 69:43-51.
Cantrell MA, Anderson D, Cerretti DP, et al. (1985) Cloning, sequence, and expression of a human granulocyte/macrophage colony-stimulating factor. Proc Natl Acad Sci USA 82:6250-6254.
Cebon J, Nicola N, Ward M, et al. (1990) Granulocyte-macrophage colony stimulating factor from human lymphocytes. The effect of glycosylation on receptor binding and biological activity. J Biol Chem 265:4483-4491.
Christensen BG, Leanza WJ, Beattie TR, et al. (1969) Phosphonomycin: structure and synthesis. Science 166:123-125.
Diederichs K, Jacques S, Boone T, Karplus PA (1991) Low-resolution structure of recombinant human granulocyte-macrophage colony stimulating factor. J Mol Biol 221:55-60.
Fuschillo S, De Felice A, Balzano G (2008) Mucosal inflammation in idiopathic bronchiectasis: cellular and molecular mechanisms. Eur Respir J 31:396-406.
Gobernado M (2003) Fosfomicina. Rev Esp Quimioter 16:15-40.
de Groot RP, Coffer PJ, Koenderman L (1998) Regulation of proliferation, differentiation and survival by the IL-3/IL-5/GM-CSF receptor family. Cell Signal 10:619-628.
Hartl D, Griese M, Kappler M, et al. (2005) Pulmonary T(H)2 response in Pseudomonas aeruginosa-infected patients with cystic fibrosis. J Allergy Clin Immunol 117:204-211.
Hayashida K, Kitamura T, Gorman DM, et al. (1990) Molecular cloning of a second subunit of the receptor for human granulocyte-macrophage colony-stimulating factor (GM-CSF): reconstitution of a high-affinity GM-CSF receptor. Proc Natl Acad Sci USA 87:9655-9659.
Hendlin D, Stapley EO, Jackson M, et al. (1969) Phosphonomycin, a new antibiotic produced by strains of streptomyces. Science 166:122-123.
Heslet L, Bay C, Nepper-Christensen S (2012) The immunomodulatory effect of inhaled granulocyte-macrophage colony-stimulating factor in cystic fibrosis. A new treatment paradigm. J Inflamm Res 5:19-27.
Karageorgopoulos DE, Wang R, Yu XH, Falagas ME (2012) Fosfomycin: evaluation of the published evidence on the emergence of antimicrobial resistance in Gram-negative pathogens. J Antimicrob Chemother 67:255-268.
Kaushansky K, O'Hara PJ, Berkner K, et al. (1986) Genomic cloning, characterization, and multilineage growth-promoting activity of human granulocyte-macrophage colony-stimulating factor. Proc Natl Acad Sci USA 83:3101-3105.
Kitamura T, Hayashida K, Sakamaki K, et al. (1991) Reconstitution of functional receptors for human granulocyte/macrophage colony-stimulating factor (GM-CSF): evidence that the protein encoded by the AIC2B cDNA is a subunit of the murine GM-CSF receptor. Proc Natl Acad Sci USA 88:5082-5086.
LeVine AM, Reed JA, Kurak KE, et al. (2005) GM-CSF-deficient mice are susceptible to pulmonary group B streptococcal infection. Pediatr Pulmonol Suppl 28:(239) 272.
Lopez AF, Vadas MA, Woodcock JM, et al. (1991) Interleukin-5, interleukin-3, and granulocyte-macrophage colony-stimulating factor cross-compete for binding to cell surface receptors on human eosinophils. J Biol Chem 266:24741-24747.
Marier JF, Brazier JL, Lavigne J, et al. (2003) Liposomal tobramycin against pulmonary infections of Pseudomonas aeruginosa: a pharmacokinetic and efficacy study following single and multiple intratracheal administrations in rats. J Antomicrob Chemother 52:247-252.
Martinez-Garcia MA, Soler-Cataluña JJ, Sanz YD, et al. (2011) Factors associated with bronchiectasis in patients with COPD. Chest 1405:1130-1137.
Meers P, Neville M, Malinin V, et al. (2008) Biofilm penetration, triggered release and in vivo activity of inhaled liposomal amikacin in chronic Pseudomonas aeruginosa lung infections. J Antimicrob Chemother 61:859-868.
Moonen P, Mermod JJ, Ernst JF, et al. (1987) Increased biological activity of deglycosylated recombinant human granulocyte/macrophage colony-stimulating factor produced by yeast or animal cells. Proc Natl Acad Sci USA 84:4428-4431.
Moser C, Kjaersgaard S, Pressler T, et al. (2000) The immune response to chronic Pseudomonas aeruginosa lung infection in cystic fibrosis is predominantly of the TH2 type. APMIS 108:329-35.
Moser C, Kobayashi O, Hougen HP, et al. (2002) Improved outcome of chronic Pseudomonas aeruginosa lung infection is associated with the induction of a TH1 dominated cytokine response. Clin Exp Immunol 127:206-213.
O'Brien C, Guest PJ, Hill SL, Stockley RA (2000) Physiological and radiological characterisation of patients diagnosed with chronic obstructive pulmonary disease in primary care. Thorax 558:635-642.
Patel IS, Vlahos I, Wilkinson TM, et al. Bronchiectasis, exacerbation indices, and inflammation in chronic obstructive pulmonary disease. Am J Respir Crit Care Med 2004 1704:400-407.
Quinton LJ (2012) GM-CSF: a double dose of protection during pneumonia. Am J Physiol Lung Cell Mol Physiol 302:L445-L446.
Robson M, Kucukcelebi A, Carp SS, et al. (1994) Effects of granulocyte-macrophage colony-stimulating factor on wound contraction. Eur J Clin Microbiol Infect Dis 3 Suppl 2:S41-S46.
Rogers GB, van der Gast CJ, Cuthbertson L, et al. (2013) Clinical measures of disease in adult non-CF bronchiectasis correlate with airway microbiota composition. Thorax 68:731-737.
Rose RM, Kobzik L, Dushay K, et al. (1992) The effect of aerosolized recombinant human granulocyte macrophage colony-stimulating factor on lung leukocytes in nonhuman primates Am Rev Respir Dis 146:1279-1286.
Sato N, Sakamaki K, Terada N, et al. (1993) Signal transduction by the high-affinity GM-CSF receptor: two distinct cytoplasmic regions of the common beta subunit responsible for different signaling. EMBO J 12:4181-4189.
Shanafelt AB, Miyajima A, Kitamura T, Kastelein RA (1991a) The amino-terminal helix of GM-CSF and IL-5 governs high affinity binding to their receptors. EMBO J 10:4105-4112.
Shanafelt AB, Johnson KE, Kastelein RA (1991b) Identification of critical amino acid residues in human and mouse granulocyte-macrophage colony-stimulating factor and their involvement in species specificity. J Biol Chem 266:13804-13810.
Smith DM, Snow DE, Rees E, et al. (2010) Evaluation of the bacterial diversity of pressure ulcers using bTEFAP pyrosequencing. BMC Med Genomics 3:41.
Standiford LR, Standiford TJ, Newstead MJ, et al. (2012) TLR4-dependent GM-CSF protects against lung injury in Gram-negative bacterial pneumonia. Am J Physiol Lung Cell Mol Physiol 302:L447-L454.
Steinwede K, Tempelhof O, Bolte K, et al. (2011) Local delivery of GM-CSF protects mice from lethal pneumococcal pneumonia. J Immunol 187(10):5346-5359.
Szoka F Jr, Papahadjopoulos D (1980) Comparative properties and methods of preparation of lipid vesicles (liposomes). Annu Rev Biophys Bioeng 9:467-508.
Wong GG, Witek JS, Temple PA, et al. (1985) Human GM-CSF: molecular cloning of the complementary DNA and purification of the natural and recombinant proteins. Science 228:810-815.

## Claims

1. A pharmaceutical composition comprising:
a. granulocyte-macrophage colony-stimulating factor (GM-CSF) in the form of molgramostim or sargramostim or regramostim, and
b. fosfomycin in the form of an inorganic or organic salt thereof
for use in the treatment or alleviation of bacterial lung infections or colonization and formulated for administration via the airways by inhalation or by intratracheal or intrabronchial or intraalveolar administration.

2. The pharmaceutical composition for use in a treatment or alleviation according to claim 1, wherein the composition is for inhalation as a powder or as an aerosol.

3. The pharmaceutical composition for use in a treatment or alleviation according to any one of claims 1-2, wherein the GM-CSF and/or the fosfomycin salt and or any additional antibiotic or antimicrobial agent are formulated as powder to be reconstituted with water as solution for nebulization.

4. The pharmaceutical composition for use in a treatment or alleviation according to any one of the preceding claims, comprising GM-CSF at a concentration of 50 micrograms per milliliter to 1000 micrograms per milliliter.

5. The pharmaceutical composition for use in a treatment or alleviation according to any one of the preceding claims, wherein at least 200 micrograms, or at least 250 micrograms or at least 300 micrograms of GM-CSF is administered per day.

6. The pharmaceutical composition for use in a treatment or alleviation according to any one of the preceding claims, wherein the composition is made for administration of GM-CSF in an amount of from 25 micrograms to 750 micrograms per dose.

7. The pharmaceutical composition for use in a treatment or alleviation according to any one of the preceding claims, wherein the composition is made for administration of from 20 milligrams to 1 gram of fosfomycin per dose, such as from 40 milligrams to 500 milligrams of fosfomycin per dose.

8. The pharmaceutical composition for use in a treatment or alleviation according to any one of the preceding claims, wherein the composition is made for administration of a dosage comprising from 25 micrograms to 750 micrograms of GM-CSF and from 20 milligrams to 1 gram of fosfomycin, such as from 40 milligrams to 500 milligrams of fosfomycin.
